## Europäisches Patentamt
## European Patent Office
## Office européen des brevets

(19)

(11) Veröffentlichungsnummer: **0 150 317**
**B1**

(12)

# EUROPÄISCHE PATENTSCHRIFT

(45) Veröffentlichungstag der Patentschrift:
18.03.87

(51) Int. Cl.⁴: **C 07 C 21/067**, C 07 C 17/10

(21) Anmeldenummer: 84114293.8

(22) Anmeldetag: 27.11.84

(54) Verfahren zur Herstellung von Methallylchlorid.

(30) Priorität: 25.01.84 DE 3402446

(43) Veröffentlichungstag der Anmeldung:
07.08.85 Patentblatt 85/32

(45) Bekanntmachung des Hinweises auf die Patenterteilung:
18.03.87 Patentblatt 87/12

(84) Benannte Vertragsstaaten:
AT BE CH DE FR GB IT LI NL SE

(56) Entgegenhaltungen:
DE-B-1 281 430

INDUSTRIAL AND ENGINEERING CHEMISTRY,
Band 31, 1939, Easton. J. BURGIN et al.:
"Halogenation of Hydrocarbons. Chlorination of
olefins containing an unsaturated tertiary carbon
atom", Seiten 1413-1419
CHEMISCHE TECHNIK, 9. Jahrgang, Heft 9,
September 1957. A. STRIEGLER: "Erfahrungen bei
der Chlorierung von Isobutylen", Seiten 523-529

(73) Patentinhaber: HÜLS AKTIENGESELLSCHAFT,
Patentabteilung / PB 15 - Postfach 13 20, D-4370
Marl 1 (DE)

(72) Erfinder: Sticken, Gerhard, Dr., Am Gecksbach 32,
D-4270 Dorsten 11 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 150 317 B1

**Beschreibung**

Methallylchlorid (3-Chlor-2-methyl-1-propen) wird technisch durch Umsetzen von Isobuten mit Chlor in der Gasphase hergestellt. Die Reaktion läuft in einem gekühlten Rohrreaktor bei Temperaturen um (möglichst unter) 100 °C und bei etwa Atmosphärendruck ab. Die erforderliche Reaktionszeit liegt im Bereich von 0,5 bis zu wenigen Sekunden. Zur Vermeidung der Weiterchlorierung des Methallylchlorids wird durchweg mit Isobutenüberschuß gearbeitet. Die Einsatzstoffe werden dem Reaktor über eine Zweistoffdüse zugeführt. Die Verdüsung von flüssigem Isobuten als indirektes Kühlmittel hat sich nicht bewährt; sie führt zu verstärkter Bildung unerwünschter, höherchlorierter Produkte, was offenbar eine Folge der schlechten Vermischung der Reaktionspartner ist (Ullmanns Encyklopädie der technischen Chemie, 4. Auflage, Band 9, S. 472 ff., Verlag Chemie, Weinheim 1975).

Nach Versuchen von A. Striegler ("Erfahrungen bei der Chlorierung von Isobutylen", Chem. Techn. 9, 9. Jg., 1957, S. 523 ff.) treten bei der Umsetzung von Isobuten mit Chlor im Labormaßstab Unregelmäßigkeiten auf, die sich durch einen plötzlichen, unerklärlichen Temperaturanstieg (von 70 °C auf 100 bis 140 °C) bemerkbar machen und eine deutliche Zunahme der unerwünschten, höherchlorierten Produkte zur Folge haben (Kap. 2.3, S. 525). Wegen der schwierigen Vermischung der Reaktionspartner in einer "halbtechnischen" Versuchsanlage wird die technische Realisierung der Gasphasenchlorierung sogar ganz in Frage gestellt: "Es gelang nicht, die unkontrollierbaren Schwankungen in der Zusammensetzung des Chlorierungsproduktes zu erklären und zu beheben" (Kap. 2.45, S. 526/527).

Obwohl die großtechnische Isobutenchlorierung inzwischen trotz des warnenden Literaturhinweises realisiert werden konnte, sind aufgrund eigener Erfahrungen mit diesem Reaktionssystem auch im technischen Maßstab die beschriebenen Unregelmäßigkeiten zu beobachten und zwar mit wechselnder Häufigkeit und Dauer. Nicht selten steigt die Reaktionstemperatur innerhalb weniger Minuten auf Werte von 300 °C an, wobei starke Rußbildung einsetzt.

Die gleichmäßige Verdüsung der Einsatzstoffe kann dann nicht mehr gewährleistet werden, die Anlage muß abgestellt und gereinigt werden.

Infolge unkontrollierbarer Unregelmäßigkeiten bei den Verfahren des Standes der Technik ergab sich die Aufgabe, ein Verfahren zu finden, das entgegen der bisherigen Lehre auch großtechnisch störungsfrei bei weitgehend konstanter Temperatur durchgeführt werden kann.

Diese Aufgabe wird erfindungsgemäß entsprechend den Angaben der Patentansprüche gelöst.

Die Zugabe von Sauerstoff (in Form von Luft) wurde zwar auch schon von A. Striegler (s. o.) versuchsweise betrieben; dieser stellte jedoch lediglich fest, daß er nicht stört (Kap. 2.34, S. 526 oben). Dieser Befund ist überdies einer Arbeit von J. Burgin et al. aus dem Jahre 1939 zu entnehmen (Ind. Eng. Chem. 31 (1939), S. 1413-1419); dort ist auf Seite 1416 rechte Spalte oben zu lesen: "The absence of any effect of even large amounts of oxygen upon the chlorination of isobutene under the above conditions is particulary significant." Lediglich bei Chlorierungen unter Licht-Katalyse nimmt Sauerstoff Einfluß, er inhibiert nämlich den Reaktionsablauf, wie unmittelbar anschließend festgestellt wird.

Schließlich wird der Einsatz von Sauerstoff erwähnt in der DE-B-1 281 430 "Verfahren zur Herstellung von Methallylchlorid". Bei diesem Verfahren wird ein Isobutylen enthaltendes Gemisch von $C_4$-Kohlenwasserstoffen mit Chlor im Temperaturbereich -50 bis +60 °C umgesetzt. Hier hat der Sauerstoff die Aufgabe, die Chlorierung der (gesättigten) Paraffinkohlenwasserstoffe zu begrenzen (Spalte 5, Z. 24-37).

Keinem der nach diesem Stand der Technik bekannten Verfahren zur Herstellung von Methallylchlorid ist zu entnehmen, daß Sauerstoff in bestimmten ausgewählten Mengen ein hervorragendes Mittel darstellt, um vorerwähnte Unregelmäßigkeiten bei der Umsetzung von Isobuten, das keine Paraffinkohlenwasserstoffe enthält, mit Chlor in Abwesenheit von Licht als Katalysator zu beseitigen.

Das erfindungsgemäße Verfahren bezieht sich auf die Reaktion von Isobuten, das keine Paraffinkohlenwasserstoff enthält, mit Chlor zur Herstellung von Methallylchlorid in der Gasphase und in Abwesenheit von Licht als Katalysator.

Der Einsatz von Sauerstoff zur Stabilisierung der Gasphasenchlorierung von Isobuten bringt folgende Vorteile: Keine Ausfallzeiten der Fabrikationsanlage mehr - konstant hohe Ausbeuten an Methallylchlorid durch Gewährleistung konstant niedriger Reaktionstemperaturen. Dies gilt insbesondere dann, wenn die Reaktion bei leicht erhöhtem Druck gefahren wird (bis etwa 3 bar (abs.), denn eigene Erfahrungen haben gezeigt, daß bei leicht erhöhtem Druck die sonst bei Normaldruck durchgeführte Umsetzung besonders häufig erhöhte Reaktionstemperaturen und damit verringerte Methallylchloridausbeuten aufweist.

Die anzuwendende Sauerstoffmenge hängt von der Reinheit der verwendeten Einsatzstoffe ab. Gehalte zwischen 0,001 und 1 Vol.-% Sauerstoff, bezogen auf die Summe der gasförmigen Einsatzstoffe, reichen aus, um ein stabiles Reaktionsgeschehen zu gewährleisten. In den meisten Fällen kann mit einer Sauerstoffmenge von < 1 Vol.-% befriedigend gearbeitet werden. Bevorzugt setzt man 0,01 bis 0,5 Vol.-% Sauerstoff ein. Sauerstoffmengen über 1 Vol.-% haben den Nachteil, daß ihre Entfernung zur Produktverlusten führt.

Man setzt den Sauerstoff als solchen oder in Form von Luft ein.

Wesentlich für die Durchführung ist die gute Vermischung des Sauerstoffs mit dem Reaktionsgemisch, die zweckmäßigerweise vor Reaktionsbeginn zu erfolgen hat. Je besser diese Vermischung ist, umso weniger Sauerstoff wird benötigt.

Selbstverständlich ist es auch möglich, das erfindungsgemäße Verfahren z. B. mit Chlor zu betreiben, welches entsprechend seiner Herstellung bzw. Aufarbeitung bereits Sauerstoff in ausreichender Menge enthält (z. B. sogenanntes Restchlor); in einem solchen Fall erübrigt sich natürlich die separate Sauerstoffeinspeisung.

Weiterhin wurde gefunden, daß in einigen speziellen Fällen auch Chlorwasserstoff als Reaktionsstabilisator eingesetzt werden kann, insbesondere dann, wenn das benützte Chlor Dichlormonoxid ($Cl_2O$) als Verunreinigung enthält. Die erforderliche Chlorwasserstoffmenge liegt bei 0,01 bis 5 Vol.-%, bevorzugt unter 1 Vol.-%, bezogen auf die Summe der gasförmigen Einsatzstoffe. Man kann auch eine Mischung von Sauerstoff und Chlorwasserstoff einsetzen.

Die nachfolgenden Beispiele erläutern das erfindungsgemäße Verfahren.

## Beispiel 1

In einem mantelgekühlten Rohrreaktor werden Isobuten und Chlor im Molverhältnis 1,04 : 1 zu Methallylchlorid als Hauptreaktionsprodukt umgesetzt. Die Reaktionsteilnehmer werden dem Reaktor über eine Zweistoffdüse zugeführt. Der Druck liegt im Mittel bei 1,6 bar (abs.). Die Reaktionstemperatur wird nach 4,8 und 12 m Reaktionsstrecke gemessen (Meßstellen $T_4$, $T_8$ und $T_{12}$). Die Reaktion wird mit 0,07 Vol.-% Luft entsprechend 0,013 Vol.-% Sauerstoff "stabilisiert".

Zur Demonstration der stabilisierenden Wirkung des zugespeisten Sauerstoffs wurde die Sauerstoffeinspeisung versuchsweise unterbrochen. Daraufhin stieg die Reaktionstemperatur nach 4 m Reaktionsstrecke innerhalb von 4 Minuten von 94 auf 220 °C an. Der Anstieg verlief nahezu linear. Das Wiederzuspeisen von Sauerstoff in der ursprünglichen Menge führte innerhalb von etwa 10 Minuten zu einer Wiederherstellung des ursprünglichen Temperaturniveaus.

Ein ähnliches Ausbleiben der Sauerstoffzuspeisung im Störungsfall der Anlage führte zum "Durchgehen" der Reaktion; die Reaktionstemperatur erreichte Werte von über 400 °C, und starke Rußbildung setzte ein; die Anlage mußte abgestellt werden.

Die nachfolgende Tabelle gibt einen Überblick über den Temperaturverlauf bei vorerwähnter versuchsweiser Unterbrechung der Sauerstoffzufuhr.

| Zeit | Sauerstoffgehalt des Gaseinsatzgemisches | Temperaturen | | |
|---|---|---|---|---|
| | | $T_4$ | $T_8$ | $T_{12}$ |
| min | Vol.-% | °C | °C | °C |
| - 10 | 0,013 | 94 | 88 | 64 |
| 0 | 0,000 | 94 | 88 | 64 |
| 1 | 0,000 | 113 | 94 | 66 |
| 2 | 0,000 | 150 | 100 | 67 |
| 3 | 0,000 | 190 | 104 | 68 |
| 4 | 0,013 | 218 | 106 | 69 |
| 5 | 0,013 | 195 | 98 | 66 |
| 6 | 0,013 | 135 | 90 | 64 |
| 8 | 0,013 | 113 | 89 | 64 |
| 13 | 0,013 | 94 | 88 | 63 |

## Beispiel 2

Die Herstellung von Methallylchlorid wird wie im Beispiel 1 beschrieben durchgeführt, jedoch mit anderem Sauerstoffgehalt und zeitweise ohne besondere Vermischung.

Die zudosierte Luftmenge liegt bei 0,44 Vol.-% entsprechend 0,088 Vol.-% Sauerstoff, bezogen auf die Summe der gasförmigen Einsatzstoffe; die Zugabe erfolgt ohne besondere Vermischungsvorkehrungen auf der Chlorseite vor der Zweistoffdüse.

Zur Überprüfung der zur Stabilisierung der Reaktion notwendigen Sauerstoffmenge wird die Luftzugabemenge halbiert. Daraufhin steigt die Reaktionstemperatur nach 4 m Reaktionsstrecke innerhalb von etwa 5 Minuten von 88 auf 141 °C. Die Verfolgung des Temperaturgeschehens über weitere 15 Minuten zeigt, daß die Reaktion offenbar auch unter diesen Bedingungen gerade noch stabil gefahren werden kann. Die Analyse des Reaktionsgemisches zeigt indes einen Abfall der Methallylchloridausbeute (bezogen auf die nach der Reaktionsgleichung $C_4H_8 + Cl_2 \rightarrow C_4H_7Cl + HCl$ stöchiometrisch erforderlichen Isobutenmenge) von 85 auf 80 bis 82 %.

Unmittelbar im Anschluß an diesen Test wurde versucht, die verminderte Luftmenge besonders gut mit dem Chlorstrom zu vermischen. Dazu wurde die Einströmgeschwindigkeit der Luft in das Chlor durch Vergrößerung des Druckabfalls zwischen Zuluft und Chlor am Zuspeiseventil erhöht; während zunächst das Entspannungsverhältnis bewußt niedrig gehalten wurde mit einem Wert von etwa 1,1, erfolgte nunmehr eine Anhebung auf Werte zwischen 1,5 und 2,0. Es zeigte sich, daß diese Verbesserung der Vermischung des Reaktionsstabilisators mit den Einsatzgasen schon ausreichte, um die Rückkehr der Temperaturverhältnisse auf die Niveaus zu veranlassen, die bei Einsatz der doppelten Luftmenge vorlagen, was wiederum die Wiederherstellung der ursprünglichen (höheren) Methallylchloridausbeute zur Folge hatte.

Die nachfolgende Tabelle gibt einen Überblick über die erhaltenen Meßdaten:

| Zeit | Sauerstoff-Zuspeisung | | Temperaturen | | |
|---|---|---|---|---|---|
| | Art | Gehalt *) | $T_4$ | $T_8$ | $T_{12}$ |
| min | | Vol.-% | °C | °C | °C |
| - 10 | "unverdüst" | 0,088 | 88 | 89 | 75 |
| 0 | "unverdüst" | 0,044 | 88 | 89 | 75 |
| 2 | "unverdüst" | 0,044 | 124 | 102 | 76 |
| 3 | "unverdüst" | 0,044 | 135 | 105 | 76 |
| 4 | "unverdüst" | 0,044 | 140 | 106 | 77 |
| 5 | "unverdüst" | 0,044 | 142 | 107 | 77 |
| 10 | "unverdüst" | 0,044 | 141 | 106 | 76 |
| 20 | "verdüst" | 0,044 | 141 | 106 | 76 |
| 21 | "verdüst" | 0,044 | 115 | 100 | 76 |
| 23 | "verdüst" | 0,044 | 98 | 92 | 76 |
| 25 | "verdüst" | 0,044 | 91 | 90 | 75 |
| 27 | "verdüst" | 0,044 | 88 | 89 | 75 |
| 29 | "verdüst" | 0,044 | 87 | 89 | 75 |
| 35 | "verdüst" | 0,044 | 86 | 89 | 74 |

*) bezogen auf den Gesamteinsatz und zugegeben in Form von Luft

**Beispiel 3**

Die Herstellung von Methallylchlorid aus Isobuten und Chlor in der Gasphase wird nach den Angaben des Beispiels 1 betrieben, jedoch zu einem anderen Zeitpunkt mit anderen Sauerstoffanteilen.

Es gelangt Chlor aus einer Diaphragma-Elektrolyse zum Einsatz. Diesem wird Sauerstoff in Form von Luft als Reaktionsstabilisator zugesetzt; der Anteil, bezogen auf den Gesamteinsatz, beträgt 1,1 Vol.-%. Die Reaktionstemperatur nach 4 m Reaktionsstrecke liegt bei 103 °C.

Diese "Normalfahrweise" wird versuchsweise unterbrochen. Statt Luft wird Chlorwasserstoff zugegeben, und zwar in einer Menge von 0,34 Vol.-%, bezogen auf den Gesamteinsatz. Es zeigt sich keine Veränderung im Reaktionsverhalten.

Die sich anschließende, versuchsweise Abstellung der Chlorwasserstoffzuspeisung führt innerhalb von 2 Minuten zu einem nahezu linearen Anstieg der Reaktionstemperatur auf 140 °C, womit das "Durchgehen" der Reaktion bei weiterem Ausbleiben von Chlorwasserstoff als Reaktionsstabilisator eindeutig angezeigt wird. Die daraufhin erfolgte Wiederzuspeisung von Chlorwasserstoff bewirkt eine Wiederherstellung des ursprünglichen Reaktionszustandes innerhalb von etwa 15 Minuten.

Alsdann wird der Chlorwasserstoffanteil von 0,34 auf 0,12 Vol.-% erniedrigt. Als unmittelbare Folge kann ein (vergleichsweise langsamer) Anstieg der Reaktionstemperatur von 103 auf 110 bis 115 °C beobachtet werden. Der Reaktionszustand bleibt auf diesem Temperaturniveau mindestens 20 Stunden lang stabil.

**Beispiel 4**

Methallylchlorid wird aus Isobuten und Chlor in der Gasphase nach den Angaben des Beispiels 1 hergestellt, jedoch erfolgt die Sauerstoffeinspeisung nunmehr in Form reinen Sauerstoffs in einer Menge von 0,020 Vol.-%, bezogen auf den Gesamteinsatz.

Das herrschende Temperaturniveau ist nahezu identisch mit dem in Beispiel 1 ausgewiesenen.

Die versuchsweise Unterbrechung der Sauerstoffeinspeisung führt zu einem sofortigen Anstieg der Reaktionstemperatur. Nach 5minütiger Fahrweise ohne Sauerstoffzudosierung weist die Meßstelle $T_4$ eine Temperatur von knapp über 200 °C aus. Die Wiederzugabe von Sauerstoff in der vorerwähnten Menge zu diesem Zeitpunkt bewirkt eine Rückkehr des Temperaturniveaus auf seinen ursprünglichen Zustand innerhalb von 10 bis 15 Minuten.

Der Temperaturverlauf im einzelnen differiert so wenig von dem in Beispiel 1 ausgewiesenen, daß hier auf eine erneute Darstellung desselben verzichtet und auf den im Beispiel 1 tabellierten verwiesen wird.

**Patentansprüche**

1. Verfahren zur Herstellung von Methallylchlorid aus Isobuten und Chlor in der Gasphase unter Ausschluß von Licht und von Paraffinkohlenwasserstoffen, dadurch gekennzeichnet, daß man den gasförmigen Einsatzstoffen 0,001 bis 1 Vol.-% Sauerstoff und/oder 0,01 bis 5 Vol.-% Chlorwasserstoff zugibt.

2. Verfahren nach Anspruch 1, dadurch gekennzeichnet, daß man den Sauerstoffgehalt der gasförmigen Einsatzstoffe auf 0,01 bis 0,5 Vol.-% einstellt.

3. Verfahren nach den Ansprüchen 1 und 2, dadurch gekennzeichnet, daß man den benötigten Sauerstoff in Form von Luft zudosiert.

**Claims**

1. A process for the production of methallyl chloride from isobutene and chlorine in the gas phase with exclusion of light and in the absence of paraffin hydrocarbons, characterized in that 0.001 to 1% by volume of oxygen and/or 0.01 to 5% by volume of hydrogen chloride are added to the gaseous feed materials.

2. A process according to claim 1, characterized in that the oxygen content of the gaseous feed materials is adjusted to 0.01 to 0.5% by volume.

3. A process according to claim 1 or 2, characterized in that the required oxygen is fed in the form of air.

**Revendications**

1. Procédé de préparation de chlorure de méthallyle à partir d'isobutène et de chlore, en phase gazeuse, à l'abri de la lumière et d'hydrocarbures paraffiniques, caractérisé par le fait que l'on ajoute aux substances gazeuses mises en oeuvre de 0,001 à 1 % en volume d'oxygène et/ ou de 0,01 à 5 % en volume de gaz chlorhydrique.

2. Procédé selon la revendication 1, caractérisé par le fait que l'on ajuste, à une valeur de 0,01 à 0,5 % en volume, la teneur en oxygène des substances gazeuses utilisées.

3. Procédé selon les revendications 1 et 2, caractérisé par le fait que l'on ajoute sous forme d'air l'oxygène nécessaire.